# NEW EUROPEAN PATENT SPECIFICATION

(11) **EP 0 216 966 B2**
(45) Date of publication and mention of the opposition decision: **06.03.1996**
(45) Mention of the grant of the patent: 14.06.1989
(21) Application number: 85306281.8
(22) Date of filing: 04.09.1985
(51) Int. Cl.: C07C 231/02, C07C 233/09

(54) **Improved process for the production of N-substituted (meth) acrylamides from (meth) acrylates & amines over a metal oxide or alkoxide catalyst**
Verfahren zur Produktion von (Meth)acrylamiden aus (Meth)acrylaten und Amine über Metalloxid- oder Alkoxid-Katalysator
Procédé pour la production de (méth) acrylamines à partir de (méth) acrylates et d'amines sur un catalyseur d'oxyde ou d'alkoxyde de métal

(43) Date of publication of application: 08.04.1987
(73) Proprietor: HUNTSMAN CORPORATION, Austin, Texas 78761 (US)
(72) Inventor: McEntire, Edward Enns, Austin Texas 78753 (US); Knifton, John Frederick, Austin Texas 78750 (US); Yit Nieh, Edward Chung, Austin Texas 78759 (US); Sellstrom, Kathy Beth, Austin Texas 78727 (US)
(74) Representative: Winkler, Andreas, Dr.

(56) References cited:
- EP-A- 0 117 530
- DE-A- 3 048 020
- DE-A- 3 123 970
- GB-A- 2 075 495
- GB-A- 2 100 732

## Description

The invention is related to a catalytic method for the production of N-substituted (meth) acrylamides and is more particularly related to a catalytic method for the production of N-substituted (meth) acrylamides from (meth) acrylates and amines in the presence of an alkoxide of silicon, titanium or zirconium.

U.S. Patent 4,206,143 (corresponding to British Patent Application 2,021,101A) reveals that dialkyl stannic oxide catalysts, such as dibutyl stannic oxide, are effective for the preparation of N-substituted acrylamides and methacrylamides from the reaction of an alkyl ester of acrylic or methacrylic acid with an aliphatic, cycloaliphatic or aromatic amine, which is a primary or secondary amine.

U. S. Patent 4,321,411, issued on March 23, 1982, relates to a process for producing an N-substituted acryl-amide or methacrylamide comprising reacting an acrylic or methacrylic acid ester with an aliphatic or aromatic amine in liquid medium in the presence of a catalytic amount of an alkyltin alkoxide such as dibutyl dimethoxytin. This patent corresponds to British Patent Application No. 2,075,495A and Japanese Kokai 81-100,749.

Tin compounds are particularly useful as catalysts for the production of dialkylaminoethyl methacrylate. For example, Japanese Kokai 76-71,010 (CA 88:121898z) teaches that dialkyltin maleate, dialkyltin mercaptide and dialkylstannanediols are useful catalysts in this regard. Dibutyltin dimethoxide was found to be effective for the catalytic synthesis of dimethylaminoethyl methacrylate (DMAEMA), as described in Japanese Kokai 77-153,912 (CA 88:137169y). Further, compounds such as (C₄H₉)₂Sn(O₂CR)₂, where R is methyl or lauryl, are catalysts for the production of dialkylaminoethyl acrylates and methacrylates, as taught in Japanese Kokai 78-34,714 (CA 89:44410a).

U. S. Patent 4,301,297 reveals that DMAEMA may be prepared in high yield by subjecting methyl methacrylate and dimethylaminoethanol to transesterification in the presence of di-n-octyl tin oxide as a catalyst. Compounds such as (C₄H₉)₂SnR₂ (where R₂ is hydrogen or -OCH₃ or O₂CCH = CHCO₂), (n-C₈H₁₇)₂SnO, (C₆H₅)₃SnOCH₃ or (C₄H₉)₃SnO₂CCH = CHC-O₂Sn(C₄H₉)₃) are effective in the manufacture of dialkylaminoethyl acrylates as seen in Japanese Kokai 78-144,522 (A 90:169290p). In addition, U.S. Patent 4,281,175 discloses that DMAEMA may be made via a number of tin catalysts such as tetrabutyltin, trioctyltin ethoxide, dibutyltin dimethoxide, dibutyltin dihydride, dibutyltin dilaurate, dibutyltin maleate, bis(tributyltin) oxide and bis (dibutylmethoxytin) oxide. See also Poller, R.C., et al., "Organotin Compounds as Transesterification Catalysts", Journal of Organometallic chemistry, 173(1979) pp. C7-C8.

This invention concerns an improvement in a process for the preparation of N-substituted acrylamides. The present invention is characterized by the step of pretreating each of the water-containing feedstocks, prior to the step of reacting the feedstocks, with a C1 to C4 tetralkoxide of silicon, titanium or zirconium, in order to substantially remove water and to thereby substantially reduce the amount of by-product formed by the Michael reaction.

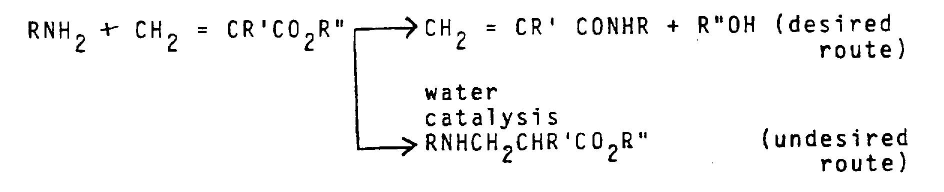

The basis process is directed to the preparation of N-substituted (meth)acrylamides of the formula: 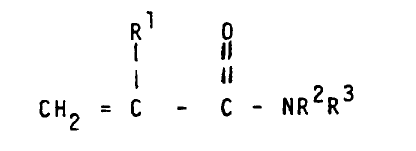 where R¹ is hydrogen or methyl, R is hydrogen or lower alkyl of 1 to 4 carbon atoms, and R³ is alkyl, aryl, alkaryl, aralkyl or alkoxyalkyl of one to twenty carbon atoms or 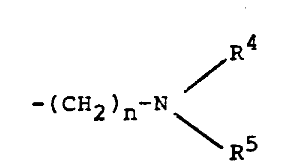 where n is an integer from 2 to 6 and R⁴ and R⁵ taken singly are lower alkyl groups to 1 to 4 carbon atoms, or R⁴ and R⁵ taken jointly are combined with the N atom to form a heterocydic ring group selected from the group consisting of morpholine, pyrrolidine or piperidine ring groups, which process comprises reacting an acrylate ester of the formula: 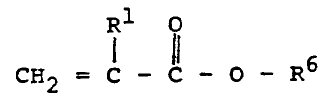 where R¹ is defined as above and R⁶ is a lower alkyl of 1 to 4 carbon atoms with an amine of the formula HNRR³ where R and R³ are defined as above over a catalytic amount of an alkyl metal oxide, an alkyl metal alkoxide, or a metal alkoxide catalyst.

The alkyl metal oxide, alkyl metal alkoxide or metal alkoxide catalyst used inthis invention may be represented by the formulae: 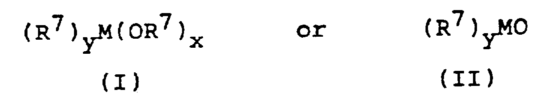 where M is a metal atom selected from the group consisting of lanthanum, niobium, tantalum, copper, zinc, tin, lead, antimony and bismuth, R⁷ is an alkyl group of one to four carbon atoms, in formule I x is 1 to 5 and y is 0 to 5 and the sum of x and y is two to five depending on the valence of the metal or, in formula II y is 1 to 3, depending on the valence of the metal.

The alkoxy groups contain alkyl substituents having one to four carbon atoms and can either be straight or branched.

The desired N-substituted acrylamides have the formula: 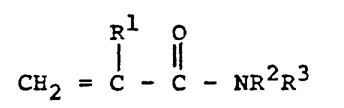 where R¹ is hydrogen or methyl, R is hydrogen or alkyl of one to four carbon atoms and R³ is alkyl, aryl, alkaryl, aralkyl or alkoxyalkyl, each of which may have one to twenty carbon atoms. The R³ group may also be: 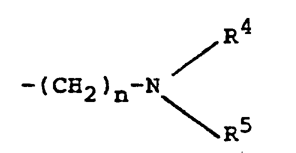 where n is an integer of from 2 to 6 and R⁴ and R⁵ when taken singly are alkyl groups containing 1 to 4 carbon atoms, or R⁴ and R⁵ when taken jointly are combined with the N atom to form a heterocyclic ring group selected from the group consisting of morpholine, pyrrolidine or piperidine ring groups. These acrylamides are formed from two reactants, an acrylate ester and an amine. A common and preferred acrylamide is dimethylaminopropylmethacrylamide (DMAPMA) which is made from methyl methacrylate (MMA) and dimethylaminopropylamine (DMAPA).

The acrylate ester has the formula: 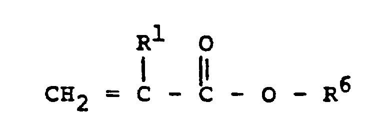 where R¹ is defined as above and R⁶ is an alkyl of one to 4 carbon atoms.

The preferred acrylates or methacrylates are methyl acrylate, ethyl acrylate, methyl methacrylate and ethyl methacrylate since these are readily accessible industrially and the alcohol liberated upon aminolysis can easily be removed from the reaction mixture. As the number of carbon atoms in the alcohol radicals increases, the suitability of the esters decreases. For that reason, the alkyl esters having more than 4 carbon atoms in the alkyl radical are considered as less preferred. Methyl acrylate and methyl methacrylate are especially preferred.

The amines useful in this invention are primary and secondary amines containing various substituents. These amines are represented by the formula HNRR³ where R and R³ are defined as above. Examples of specific amines which would be suitable are butylamine, 2-octylamine, benzylamine, dimethylaminoneopentanamine, 3-dimethyl-aminopropylamine, 2-dibutylaminoethylamine, 4-(aminopropyl)morpholine, diethylaminopropylamine, 2-dimethylamino-ethylamine, 1-(aminopropyl)piperidine, 4-(aminoethyl)morpholine. A preferred compound is 3-dimethylaminopropylamine.

The reaction should be conducted at a temperature in the range of 70 to 130°C. The preferable temperature range is 90 to 120°C. Reaction pressure should be approximately atmospheric. Reduced pressures are used as required to keep the temperature within the desired range to avoid polymerization. Increased pressure may be used with low boiling reagents. The mole ratio of amine to acrylate ester should range from 1:1 to 1:100. The amine may be added gradually during the reaction to maintain a desired mole ratio of amine to ester. The mole ratio of amine to catalyst should range from 1:0.8 to 1:0.001. As will be demonstrated, the alcohol by-product need not be removed during the reaction which would permit continuous processing, a feature not seen in many previous methods. Batch processing could, of course, be used in connection with the inventive method. The use of inhibitors to prevent the polymerization of the desired acrylamide product may also be desired. Such inhibitors include phenothiazine, N,N'-diphenylphenylenediamine, p-methoxyphenol, etc.

It has been discovered in accordance with the present invention that the alkoxides of silicon, titanium, or zirconium are very effective drying agents for amines such as DMAPA, as well as for esters such as methylmethacrylate. In accordance with the present invention the alkoxide drying agent is added in approximately stoichiometric amounts according to the water present in the reagent to be dried. We have discovered that the drying is very rapid, that no solid residue is obtained, and that the reagents may be used directly for (meth)acrylamide formation. By removing water, the drying agents significantly improve the selectivity of this reaction because of the suppression of the Michael reaction which requires the presence of water as a coreactant. However, the drying agents do not substantially adversely affect the selectivity of the (meth)acrylamide formation reaction.

The alkoxide group of the drying agent of the present invention may suitably be methoxide, ethoxide, propoxide, isopropoxide, n-butoxide, sec-butoxide or isobutoxide.

The alkoxide drying agents of the present invention are added to the reactants prior to their introduction into the reactor. Once mixed, the alkoxide agents rapidly dry the feedstock without agitation. The drying agents of this invention are effective at ambient temperatures but may also be used at elevated temperatures. The rate of drying may be improved by heating.

The alkoxide drying agents of the present invention react with water by hydrolysis. They are thus destroyed by the reaction of water and release alcohols into the solution. These alcohols may be removed by distillation during the subsequent stages of the process.

### Specific Examples

### Example I (Comparative)

To a one-liter glass reactor equipped with a 2-ft (61 cm) 1-in. (2.5 cm) diameter column and a thermometer and addition funnel were charged:
400 g methyl methacrylate (MMA)
1.2 g N,N'-diphenyl-p-phenylenediamine (DPPDA)
1.2 g phenothiazine
8.4 g tetramethyl orthosilicate drying agent
5.0 g stannous methoxide catalyst

The addition funnel was charged with 136 g (3-dimethylamino)propylamine (DMAPA).

The contents of the reactor were heated under a nitrogen atmosphere to 98°C and the dropwise addition of DMAPA was begun. The methanol-MMA azeotrope was removed by distillation as it was formed during the reaction. The DMAPA was added over 1.5 hours. After 5.5 hours, the heat was removed. A total of 51 cc of overhead had collected.

Gas chromatographic analysis showed that the solution contained 42% of the desired N-(3-dimethylaminopropyl)methacrylamide (DMAPMA) and only 4.9% of the undesired Michael adduct [methyl (3(3-dimethylaminopropyl amino)-2-methyl) propionate]. The other major components were identified as 48.3% MMA and 1.9% DMAPA.

### Example II (In accordance with the invention)

Dry bottles sealed with rubber serum stoppers were filled with 100 ml of DMAPA. A nitrogen atmosphere was provided for each bottle. Drying agents were added to the DMAPA in the bottles which were then shaken to mix the contents. The bottles then remained at room temperature until the analyis of the DMAPA in each for water by a modified Karl Fisher titration. The results are presented below:

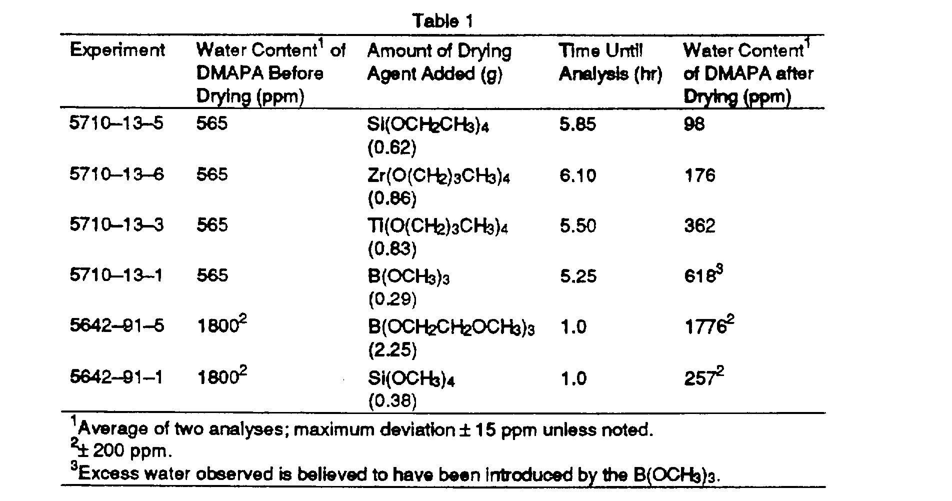

These experiments illustrate the rapid drying activity of the alkoxides of silicon, zirconium, and titanium while showing the inactivity of boron alkoxides as drying agents under these conditions.

### Example III (Comparative)

To apparatus similar to that of Example I were charged:
400 g MMA (water content 0.08%)
1.2 g DPPDA
1.2 g phenothiazine
5.0 g stannous methoxide catalyst

No drying agent was added.

The addition funnel was charged with 136 g DMAPA (water content 0.12%). The experiment was conducted exactly as Example I. Less overhead collected, however, and the experiment was terminated after 4 hours due to the slow nature of the reaction.

Gas chromatographic analysis showed that the resulting solution contained only 10.2% of the desired DMAPMA, and 14.1% of the undesired Michael adduct. The other components identified were 60.2% MMA and 14% DMAPA. Thus the water present in the feeds deactivated some of the catalyst causing the slower reaction in comparison to Example I.

### Example IV (In accordance with the invention)

To an apparatus similar to that of Example I were charged:
405 g MMA*
1.2 g DPPDA
1.2 g phenothiazine
6.43 g dibutyltin oxide catalyst

To the addition funnel was charged 137 g DMAPA** containing 2.7 g of tetramethyl orthosilicate drying agent. The DMAPA was added slowly as in Example I. At the end of 6.25 hours reaction time and a maximum reaction temperature of 121°C, the reactor contents were analyzed by gas chromatography. The product DMAPMA accounted for 50.1% whereas the Michael adduct was only 5.0% of the mixture. Other products identified were MMA (40.0%) and DMAPA (0.2%). Thus the conversion was 99.2%, and the selectivity to product was 87.7%.

*The MMA contained 774 ppm water. Before the MMA was introduced into the reactor, 2.4 g of tetraethyl orthosilicate (slightly less than one mole of drying agent per mole of water present in the MMA) was added to the MMA.

**The DMAPA contained 3472 ppm water before the addition of the drying agent. Thus one mole of tetraethyl orthosilicate was added for every 2 moles of water present.

### Example V (Comparative)

In an experiment conducted similarly to Example IV, but using no added liquid drying agent, only 84% selectivity to DMAPMA was observed at 99% DMAPA conversion. The DMAPA and MMA were predried with molecular sieves to 587 ppm and 126 ppm water, respectively.

### Example VI (In accordance with the invention)

In an experiment conducted similarly to Example IV where tetrabutylitanate was used as a drying agent instead of tetraethyl orthosilicate, 88% selectivity to DMAPMA was oserved at 99% DMAPMA conversion. The MMA containing 1424 ppm water was predried with 3.0 g tetrabutyl titanate. The DMAPA containing 2577 ppm water was dried with 3.0 g tetrabutyl titanate.

These examples illustrate the efficiency of these drying agents in the selective formation of the desired monomer. Even trace amounts of water remaining (Example V) in the reactor feeds can significantly influence the selectivity of the reaction.

## Claims

1. A process for the preparation of N-substituted (meth) acrylamides of the formula: 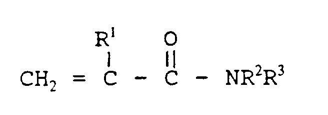 where R¹ is hydrogen or methyl, R is hydrogen or alkyl having 1 to 4 carbon atoms and R³ is alkyl, aryl, alkaryl, aralkyl or alkoxyalkyl having 1 to 20 carbon atoms or 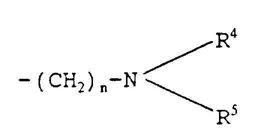 wherein n is 2 to 6, and R⁴ and R⁵, which may be the same or different are alkyl having 1 to 4 carbon atoms, or R⁴ and R⁵, together with the N atom to which they are attached, are a substituted or unsubstituted morpholine, pyrrolidine or piperidine ring,
which process comprises reacting a feedstock of an acrylate ester of the formula: 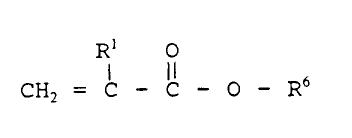 wherein R¹ has the meaning given above and R⁶ is alkyl having 1 to 4 carbon atoms, with a feedstock of an amine of the formula HNRR³, wherein R and R³ have the meanings given above, in the presence of an alkyl metal oxide, alkyl metal alkoxide or metal alkoxide catalyst having the formula:
(R⁷)_{y}M(OR⁷)ₓ (I)
or
(R⁷)_{y}MO (II)
wherein M is lanthanum, niobium, tantalum, copper, zinc, tin, lead, antimony or bismuth, R⁷ is alkyl having 1 to 4 carbon atoms, x is 1 to 5 and y is 0 to 5 and the sum of x and y is 2 to 5, depending on the valence of the metal, for formula I and y is 1 to 3 depending on the valence of the metal, for formula II,
characterised in that the said feedstocks contain water, and have been treated, prior to the step of reacting the feedstocks, with a C₁ to C₄ tetralkoxide of silicon, titanium or zirconium, as a drying agent.

2. A process according to Claim 1 characterised in that the drying agent is a silicon tetralkoxide.

3. A process according to Claim 2 characterised in that the silicon tetralkoxide is tetramethylorthosilicate or tetraethylorthosilicate.

4. A process according to Claim 1 characterised in that the drying agent is a zirconium tetralkoxide.

5. A process according to Claim 4 characterised in that the zirconium tetralkoxide is tetrabutyl orthozirconate.

6. A process according to Claim 1 characterised in that the drying agent is a titanium alkoxide.

7. A process according to Claim 6 characterised in that the titanium alkoxide is tetrabutylorthotitanate.

## Patentansprüche

1. Verfahren zur Herstellung von N-substituierten (Meth)acrylamiden der Formel 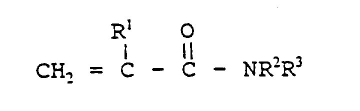 worin R¹ für Wasserstoff oder Methyl, R für Wasserstoff oder Alkyl mit 1 bis 4 Kohlenstoffatomen und R³ für Alkyl, Aryl, Alkaryl, Aralkyl oder Alkoxyalkyl mit 1 bis 20 Kohlenstoffatomen oder 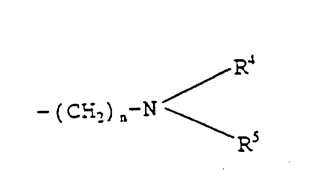 wobei n 2 bis 6 ist, und R⁴ und R⁵, die gleich oder verschieden sein können, Alkyl mit 1 bis 4 Kohlenstoffatomen bedeuten oder R⁴ und R⁵ zusammen mit dem N-Atom, an das sie gebunden sind, einen substituierten oder unsubstituierten Morpholin-, Pyrrolidin- oder Piperidinring bilden, stehen, bei dem man ein Einsatzmaterial aus einem Acrylatester der Formel 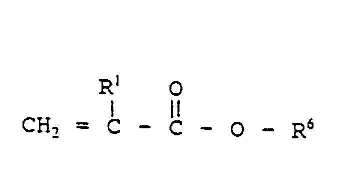 worin R¹ die oben angegebene Bedeutung hat und R⁶ für Alkyl mit 1 bis 4 Kohlenstoffatomen steht, mit einem Einsatzmaterial aus einem Amin der Formel HNRR³, wobei R und R³ die oben angegebenen Bedeutungen haben, in Gegenwart eines Alkylmetalloxid-, Alkylmetallalkoxid- oder Metallalkoxidkatalysators der Formel
(R⁷)_{y}M(OR⁷)ₓ (I)
oder
(R⁷)_{y}MO (II)
worin M Lanthan, Niob, Tantal, Kupfer, Zink, Zinn, Blei, Antimon oder Wismut und R^{⁷} für Alkyl mit 1 bis 4 Kohlenstoffatomen steht sowie bei Formel I x 1 bis 5 und y 0 bis 5 sind und die Summe von x und y je nach der Wertigkeit des Metalls 2 bis 5 beträgt und bei Formel II y je nach der Wertigkeit des Metalls 1 bis 3 ist, umsetzt, dadurch gekennzeichnet, daß die besagten Einsatzmaterialien Wasser enthalten und, vor der Stufe der Umsetzung der Einsatzmaterialien, mit einem C₁-C₄-Tetralkoxid von Silicium, Titan oder Zirkon als einem Trocknungsmittel behandelt worden sind.

2. Verfahren nach Anspruch 1, dadurch gekennzeichnet, daß das Trocknungsmittel ein Silicium-Tetralkoxid ist.

3. Verfahren nach Anspruch 2, dadurch gekennzeichnet, daß das Silicium-Tetralkoxid Tetramethyl-Orthosilicat oder Tetraethyl-Orthosilicat ist.

4. Verfahren nach Anspruch 1, dadurch gekennzeichnet, daß das Trocknungsmittel ein Zirkon-Tetralkoxid ist.

5. Verfahren nach Anspruch 4, dadurch gekennzeichnet, daß das Zirkon-Tetralkoxid Tetrabutyl-Orthozirkonat ist.

6. Verfahren nach Anspruch 1, dadurch gekennzeichnet, daß das Trocknungsmittel ein Titan-Alkoxid ist.

7. Verfahren nach Anspruch 6, dadurch gekennzeichnet, daß das Titan-Alkoxid Tetrabutyl-Orthotitanat ist.

## Revendications

1. Procédé de préparation de (méth)acrylamides N-substitués répondant à la formule 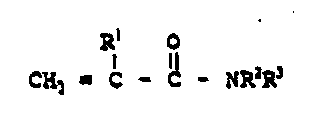 dans laquelle R¹ est un atome d'hydrogène ou un groupe méthyle, R est un atome d'hydrogène ou un groupe alkyle en C1 à C4, et R³ est un groupe alkyle, aryle, alkaryle, aralkyle ou alcoxyalkyle en C1 à C20, ou 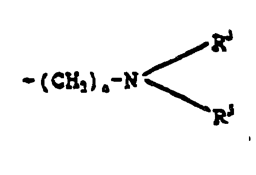 dans laquelle n est un entier de 2 à 6 et R⁴ et R^{⁵}, qui peuvent être indentiques ou différents, sont des groupes alkyle en C1 à C4, ou bien R⁴ et R⁵ conjointement avec l'atome N auquel ils sont fixés, sont un cycle morpholine, pyrrolidine ou pipéridine substitué ou non substitué, lequel procédé comprend la réaction d'une charge d'alimentation d'un ester acrylique répondant à la formule 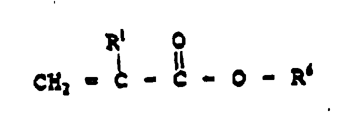 dans laquelle R¹ est tel que défini ci-dessus et R⁶ est un groupe alkyle en C1 à C4, avec une charge d'alimentation, une amine répondant à la formule HNRR³ dans laquelle R et R³ sont tels que définis ci-dessus en présence d'un oxyde de métal alkyle, d'un alcoolate de métal alkyle ou d'un alcoolate métallique comme catalyseur répondant aux formules
(R¹)_{y}M(OR⁷)ₓ (I)
or
(R⁷)_{y}MO. (II)
dans lesquelles M est le lanthane, le niobium, le tantale, le cuivre, le zinc, l'étain, le plomb, l'antimoine ou le bismuth R⁷ est un groupe alkyle en C1 à C4, x est de 1 à 5 et y est de 0 à 5 et la somme de x + y est de 2 à 5 suivant la valence du métal pour la formule (I) et y est de 1 à 3 , suivant la valence du métal pour la formule (II), caractérisé en ce que lesdites charges d'alimentation contiennent de l'eau et ont été traitées, avant le stade de réaction des charges d'alimentation par un tétraalcoolate de silicium, de titane ou de zirconium en C1 à C4.

2. Procédé suivant la revendication 1, caractérisé en ce que l'agent desséchant est un tétraalcoolate de silicium.

3. Procédé suivant la revendication 2, caractérisé en ce que le tétraalcoolate de silicium est un orthosilicate de tétraméthyle ou un orthosilicate de tétraéthyle.

4. Procédé suivant la revendication 1, caractérisé en ce que l'agent desséchant est un tétraalcoolate de zirconium;

5. Procédé suivant la revendication 4, caractérisé en ce que le tétraalcoolate de zirconium est l'orthozirconate de tétrabutyle.

6. Procédé suivant la revendication 1, caractérisé en ce que l'agent desséchant est un alcoolate de titane.

7. Procédé suivant la revendication 6, caractérisé en ce que l'alcoolate de titane est l'orthotitanate de tétrabutyle.
